Europäisches Patentamt

European Patent Office

Office européen des brevets

(11) Veröffentlichungsnummer: **0 258 690 B1**

## (12) EUROPÄISCHE PATENTSCHRIFT

(45) Veröffentlichungstag der Patentschrift: **11.03.92**

(21) Anmeldenummer: **87111559.8**

(22) Anmeldetag: **10.08.87**

(51) Int. Cl.⁵: **A61B 1/30**, A61B 5/03

(54) **Katheter zur Blasen- und Harndruckmessung.**

(30) Priorität: **01.09.86 DE 3629732**

(43) Veröffentlichungstag der Anmeldung:
**09.03.88 Patentblatt 88/10**

(45) Bekanntmachung des Hinweises auf die
Patenterteilung:
**11.03.92 Patentblatt 92/11**

(84) Benannte Vertragsstaaten:
**AT BE CH DE FR GB IT LI NL SE**

(56) Entgegenhaltungen:
**DE-A- 2 315 056**

**MEDICAL PROGRESS THROUGH TECHNOLO-
GY Band 8, Nr 2/3, 1982, Seiten 95-104, Berlin,
Deutschland; M.R. Neuman: "Physical and
Chemical Sensors for Medical Instrumentation".**

(73) Patentinhaber: **Heinz, Franz, Dr. med.
Holzweg 41
W-8036 Herrsching(DE)**

(72) Erfinder: **Heinz, Franz, Dr. med.
Holzweg 41
W-8036 Herrsching(DE)**

(74) Vertreter: **Schmidt-Evers, Jürgen, Dipl.-Ing. et
al
Patentanwälte Dipl.-Ing. H. Mitscherlich
Dipl.-Ing. K. Gunschmann Dipl.-Ing.
Dr.rer.nat. W. Körber Dipl.-Ing. J. Schmidt-
Evers Dipl.-Ing. W. Melzer Steinsdorfstrasse
10
W-8000 München 22(DE)**

**Beschreibung**

Die Erfindung betrifft einen Katheter zur Blasen- und Harnröhrendruckmessung beim Menschen, insbesondere bei Frauen, bestehend aus einem Katheterrohr mit einem ersten Druckaufnehmer an seinem in die Harnblase einzuführenden Ende und mindestens einem zweiten Druckaufnehmer in seinem mittleren Bereich, welcher nach dessen Einführen des Katheterrohres in der Harnröhre zu liegen kommt.

Katheter der hier betrachteten Art finden Anwendung zur Messung eines Harnröhrenruhe- und -streßprofiles bei harninkontinenten Menschen. Harninkontinenz ist der medizinische Begriff dafür, daß aus der Harnblase eines Menschen, insbesondere einer Frau über die Harnröhre entgegen seinem (ihrem) Willen Urin austritt. Die Inkontinenz hat im wesentlichen zwei Ursachen. Die eine Ursache ist blasenbedingt. Die andere Ursache ist verschlußbedingt. Die blasenbedingte Ursache kann medikamentös behandelt werden. Die verschlußbdingte Ursache erfordert eine operative Behandlung. Für den Arzt ist es erforderlich, herauszufinden, welche der beiden Ursachen vorliegt, bevor die Behandlung aufgenommen wird.

Um die Ursache zu ermitteln, wird der Katheter in die Harnröhre eingeführt, so daß sein vorderes Ende in die Harnblase hineinragt. Dann wird der Katheter zur Druckmessung langsam zurückgezogen. Beim Zurückziehen wird die Patientin aufgefordert, mehrfach zu husten. Durch die Hustenstöße soll einerseits impulsartig Druck auf die Blase ausgeübt werden, und andererseits soll der Schließmuskel der Harnröhre veranlaßt werden, die Schließkraft impulsartig zu erhöhen. Beide Druckaufnehmer melden dementsprechend bei Hustenstößen einen erhöhten Druck. Die von dem am Ende des Katheterrohres stammenden Druckspitzen sind während des Herausziehens des Katheterrohres etwa gleich hoch. Die von dem am mittleren Bereich des Katheterrohres befindlichen Druckaufnehmer stammenden Druckspitzen liegen auf einer gewölbten Kurve. Dies wird damit erklärt, daß die Schließkraft des Schließmuskels der Harnröhre ortsabhängig und etwa im mittleren Bereich zwischen den beiden Mündungen am größten ist. Diesem sog. Ruhedruckprofil überlagern sich dann die Druckspitzen, die nach der bisher allgemein vertretenen Auffassung ihre Ursache darin haben, daß die Schließkraft des Schließmuskels bei Hustenstößen impulsartig erhöht wird. Vergleicht man nun die durch wiederholte Hustenstöße verursachten Druckprofile der beiden Druckaufnehmer, die auch als Streßprofile bezeichnet werden, so ergibt sich nach der bisher geltenden Auffassung dann zwingend der Nachweis für eine verschlußbdingte Inkontinenz, wenn die höchsten Druckspitzen des Harnröhrenstreßprofiles, das von dem im mittleren Bereich des Katheterrohres angeordneten Druckaufnehmer stammt, niedriger sind als die entsprechenden Druckspitzen des Harnblasenstreßprofiles, das von dem am Ende des Katheterrohres befindlichen Druckaufnehmer stammt. Dies deshalb, weil dann der von der Harnblase auf den Schließmuskel wirkende Flüssigkeitsdruck größer ist als der Schließdruck des Schließmuskels.

Trotz dieser scheinbar eindeutigen physikalischen Verhältnisse mußte immer wieder festgestellt werden, daß die aus den Meßergebnissen gezogenen Schlüsse betreffend das Vorliegen einer Inkontinenz nicht mit den tatsächlichen klinischen Verhältnissen übereinstimmen. Mit anderen Worten, in Fällen wo der Harnröhrendruck den Blasendruck bei Hustenstößen überstieg, konnte klinisch Inkontinenz festgestellt werden und umgekehrt.

Nach der Druckschrift "Medical Progress Through Technology", Band 8, Nr. 2/3, 1982, S. 95-104, ist ein Katheter zur Blasen- und Harnröhrendruckmessung beim Menschen, insbesondere bei Frauen bekannt, der aus einem Katheterkörper mit einem ersten Druckaufnehmer an seinem in die Harnblase einzuführenden Ende und mindestens einem zweiten Druckaufnehmer in seinem mittleren Bereich besteht. Zwischen den beiden Druckaufnehmern ist dann im Katheterkörper ein Latex-Ballon angeordnet, dessen Aufgabe es ist, den Katheder so zu positionieren, daß der erste Druckaufnehmer nach dem Einführen des Katheterrohres in die Harnröhre in der Harnblase und der zweite Druckaufnehmer in der Harnröhre zu liegen kommt. Obwohl in der Druckschrift nicht beschrieben, ist es möglich, daß dann, wenn der Ballon gegen die innere Harnröhrenmündung gezogen wird, eine Abdichtung derselben erfolgt. Bei einer solchen Abdichtung wird der vorteilhafte Effekt erzielt, daß aufgrund der Meßergebnisse eine eindeutige Aussage über die Verschlußkräfte gemacht werden kann. Dies deshalb, weil Ursache für das Entstehen der Druckspitzen eines Harnröhrenstreßprofiles nicht allein die bei Hustenstößen impulsartig erhöhte Schließkraft des Schließmuskels der Harnröhre ist, sondern daß eine weitere Komponente hinzukommt. Diese weitere Komponente hat ihre Ursache darin, daß sich bei einem Hustenstoß die Harnröhre von ihrer inneren Mündung her kurzfristig trichterartig erweitert, wodurch unter Druck stehende Flüssigkeit (Urin oder eine in die Blase eingeführte Meßflüssigkeit) aus der Harnblase an den im mittleren Bereich des Katheterrohres befindlichen Druckaufnehmer gelangt. Wenn die Harnröhrenmündung abgedichtet wird, so wird die erwähnte weitere Komponente eliminiert.

Der erfindungsgemäße Katheter unterscheidet sich von dem bekannten Katheter dadurch, daß das Katheterrohr von einem gegenüber diesem verschiebbaren Mantelrohr umgeben ist, an dessen Ende sich der zweite Druckaufnehmer befindet. Während das Katheterrohr bei der Messung in seiner abdichtenden Stellung verbleibt, kann dann das Mantelrohr zurückgezogen werden.

Ein Vorschlag zur Weiterbildung des erfindungsgemäßen Katheters kann darin bestehen, daß am Ende des Mantelrohres in Rohrumfangsrichtung mindestens zwei Druckaufnehmer angeordnet sind. Vorzugsweise werden die beiden Druckaufnehmer diametral gegenüberliegend angeordnet. Dadurch ist es möglich, den Harnröhrendruck nicht nur an einer Stelle der Harnröhrenwandung, beispielsweise oben zu messen, sondern oben und unten. Eine derartige Messung kann von wissenschaftlichem Interesse sein, beispielsweise um eine Senkung der Harnröhrenhinterwand festzustellen.

Die Druckaufnehmer können, wie bekannt, von Microtransducern gebildet sein, deren Anschlußleitungen durch den Katheter nach außen geführten und mit Druckmeßgeräten verbindbar sind. Alternativ dazu ist es aber auch möglich, die Druckaufnehmer durch Öffnungen zu realisieren, die den Ausgang von durch den Katheter nach außen geführte Leitungen bilden, welche mit äußeren Druckmeßgeräten verbindbar sind.

Eine andere zweckmäßige Weiterbildung kann darin bestehen, daß die Öffnung den Ausgang einer durch das Mantelrohr geführten Leitung bildet, wobei die Leitung wiederum mit einer Pumpe zum Einführen von Flüssigkeit in die Harnblase verbindbar ist. Da das Mantelrohr hier zurückgezogen wird, kann über die erwähnte Öffnung bzw. Leitung zusätzlich noch Flüssigkeit in die Harnröhre eingeleitet werden, um das durch das Zurückziehen freiwerdende Volumen mit Flüssigkeit zu füllen (Perfusion).

Um stets eine sichere Abdichtung zu gewährleisten und die Messung nicht nur in sitzender Stellung der Patientin durchführen zu können, wird weiterhin vorgeschlagen, daß eine Spanneinrichtung vorgesehen ist, mittels welcher das das Abdichtmittel tragende Katheterrohr unter eine Spannung setzbar ist, die die Tendenz hat, das Katheterrohr aus der Harnblase herauszuziehen und damit das Abdichtmittel gegen die innere Harnröhrenmündung zu pressen. Die Spanneinrichtung kann beispielsweise von einer Zugfeder gebildet sein, die mit ihrem einen Ende ortsfest verankerbar ist und mit ihrem anderen Ende mit dem Katheterrohr verbunden werden kann.

Um ein zeitsteuerbares Zurückziehen des Mantelrohres gegenüber dem Katheterrohr zu gewährleisten, wird ferner vorgeschlagen, eine spezielle Rückzugseinrichtung vorzusehen, mittels welcher das Katheterrohr weitgehend ortsfest aber elastisch fixierbar ist und mittels welcher ferner das Mantelrohr motorisch entsprechend einem vorgegebenen Zeitverlauf gegenüber dem Katheterrohr in der Harnröhre zurückziehbar ist.

Ausführungsbeispiele der Erfindung werden nachfolgend anhand der Zeichnungen beschrieben. Es zeigen:

Figur 1     eine schematische Darstellung einer herkömmlichen Meßanordnung mit in die Harnröhre eingeführtem Katheter;

Figur 2a     ein Ruhedruckprofil der Harnblase (gestrichelt) und der Harnröhre (voll ausgezogen);

Figur 2b     ein Streßprofil der Harnblase (gestrichelt) und der Harnröhre (voll ausgezogen);

Figur 3     ein weiterer bekannter Katheter;

Figur 3a     einen Schnitt A-A durch Figur 3;

Figur 3b     einen Schnitt B-B durch Figur 3;

Figur 4     eine erste Ausführungsform des erfindungsgemäßen Katheters;

Figur 4a     einen Schnitt A-A durch Figur 4;

Figur 4b     einen Schnitt B-B durch Figur 4;

Figur 4c     einen Schnitt C-C durch Figur 4;

Figur 5     eine zweite Ausführungsform des erfindungsgemäßen Katheters;

Figur 5a     einen Schnitt A-A durch Figur 5;

Figur 5b     einen Schnitt B-B durch Figur 5;

Figur 6     den Katheter gemäß Figur 5 in perspektivischer Darstellung, eingeführt in die Harnröhre und Harnblase;

Figur 7     den Katheter gemäß Figur 5, eingespannt in eine Rückzugseinrichtung.

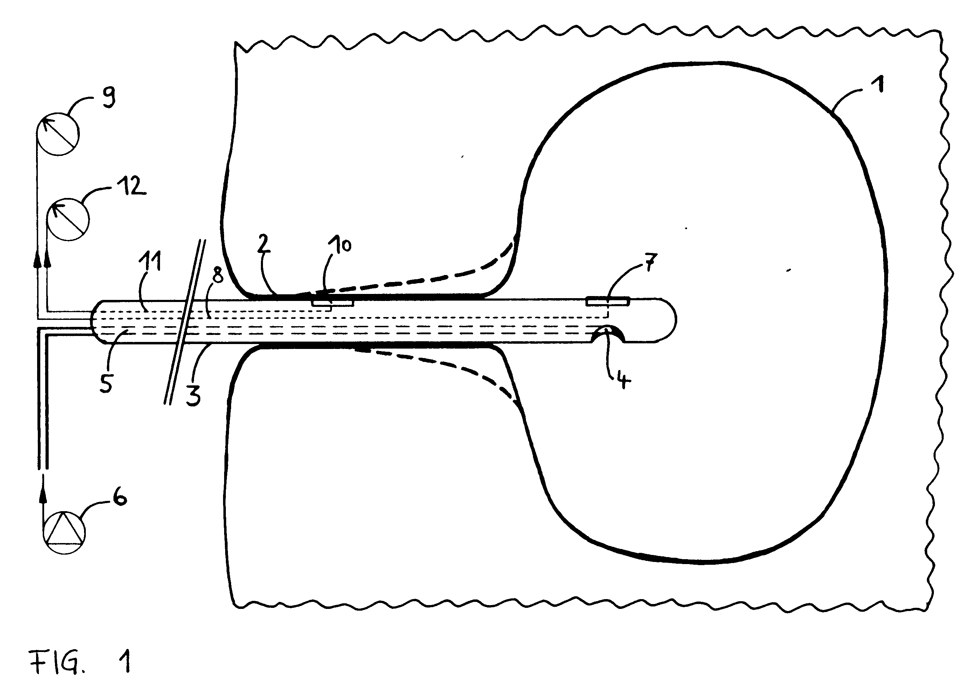

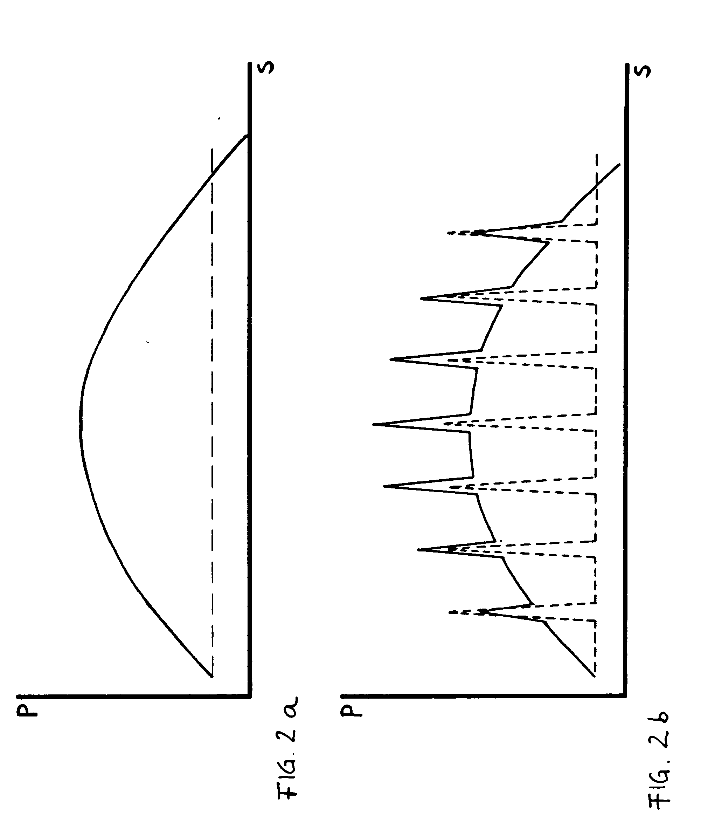

In Figur 1 sind die Harnblase 1 und die Harnröhre 2 einer Frau schematisch dargestellt. In die Harnblase 1 ist durch die Harnröhre 2 ein Katheterrohr 3 eingeführt. An dem in die Harnblase 1 eingeführten Ende des Katheterrohres 3 befindet sich eine Öffnung 4, die das Ende einer durch das Katheterrohr 3 geführten Leitung 5 bildet. Die Leitung 5 ist mit einer Pumpe 6 verbunden, von welcher aus Flüssigkeit in die Harnblase 1 eingeführt werden kann. Ferner ist an dem in die Harnblase 1 eingeführten Ende des Katheterrohres 3 ein Druckaufnehmer 7 in Form eines Microtransducers angeordnet. Der Druckaufnehmer 7 ist über eine durch das Katheterrohr 3 geführte elektrische Leitung 8 mit einem Meßgerät 9 zur Messung und Aufzeichnung des Harnblasendruckes verbunden. Weiterhin ist im mittleren Bereich des Katheterrohres 3, der in der Harnröhre 2 zu liegen kommt, ein ebenfalls als Microtransducer ausgebildeter Druckaufnehmer 10 angeordnet. Dieser ist über eine durch das Katheterrohr 3 geführte elektrische Leitung 11 mit einem Meßgerät 12 zur Messung und Aufzeichnung des Harnröhrendruckes verbunden. Wenn die Patientin hustet, so wird die innere Mündung der Harnröhre 2 erweitert, wie dies durch die gestrichelten Linien angedeutet ist, mit der Folge, daß die in der Harnblase 1 befindliche Flüssigkeit an den Druckaufnehmer 10 gelangt. Der Druckaufnehmer 10 mißt demnach bei Hustenstößen nicht nur oder sogar weniger den von dem Schließmuskel der Harnröhre 2 auf ihn ausgeübten Druckstoß, sondern auch oder im wesentlichen denjenigen, der durch die Druckerhöhung in der Harnblase 1 verursacht und von der in der Harnblase befindlichen Flüssigkeit über die trichterartige Erweiterung der Harnröhre 2 auf ihn übertragen wird.

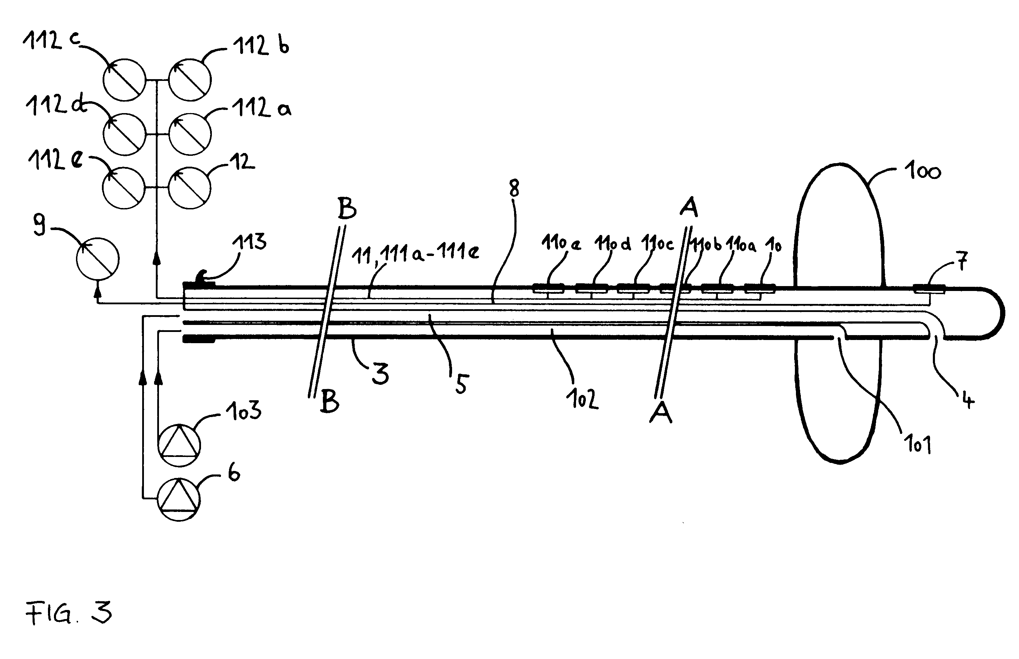

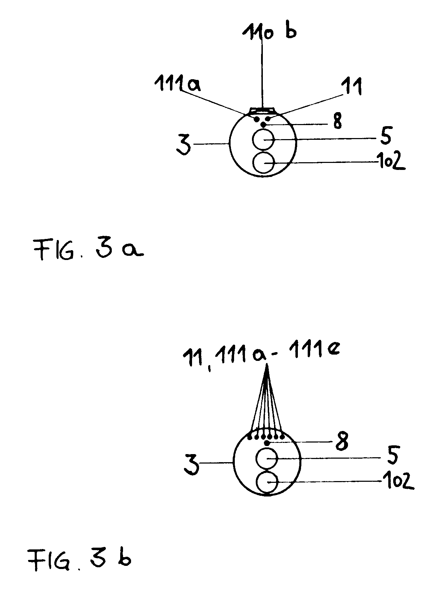

Zur Feststellung einer Inkontinenz wird das Katheterrohr 3 in der Harnröhre zurückgezogen, derart, daß der Druckaufnehmer 10 in der Harnröhre von innen nach außen wandert. In Figur 2a ist die Abhängigkeit des von dem Druckaufnehmer 10 gemessenen Druckes von dem in der Harnröhre zurückgelegten Weg bzw. der Zeit als voll ausgezogene Linie dargestellt. Vergleichsweise dazu ist der unveränderte Druck der Harnblase 1 als gestrichelte Linie dargestellt. Beide Linien werden als Ruhedruckprofil bezeichnet.

Figur 2b zeigt die Abhängigkeit des von den beiden Druckaufnehmern gemessene Druckes wiederum vom Ort des Druckaufnehmers 10 in der Harnröhre bzw. von der Zeit, wenn die Patientin veranlaßt wird, wiederholt zu husten. Die Hustenstöße verursachen an beiden Druckaufnehmern Druckstöße. Die Kurven in Figur 2b werden als Harnröhrenstreßprofil (voll ausgezogen) bzw. Harnblasenstreßprofil (gestrichelt) bezeichnet.

Wie eingangs beschrieben, muß vermieden werden, daß der Druckaufnehmer 10 in der Harnröhre 2 mit Druckstößen der in der Harnblase 1 befindlichen Flüssigkeit beaufschlagt wird. Wenn dies gelingt, so liegt gemäß Figur 2b dann keine Inkontinenz vor, wenn die höchsten Druckspitzen des Harnröhrenstreßprofiles (voll ausgezogene Linie) über den höchsten Druckspitzen des Harnblasenstreßprofiles (gestrichelte Linie) liegen. Im umgekehrten Falle ist die Schließkraft des Schließmuskels der Harnröhre geringer als die von der Flüssigkeit in der Harnblase ausgeübte Druckkraft, mit der Folge, daß eine Inkontinenz auftritt.

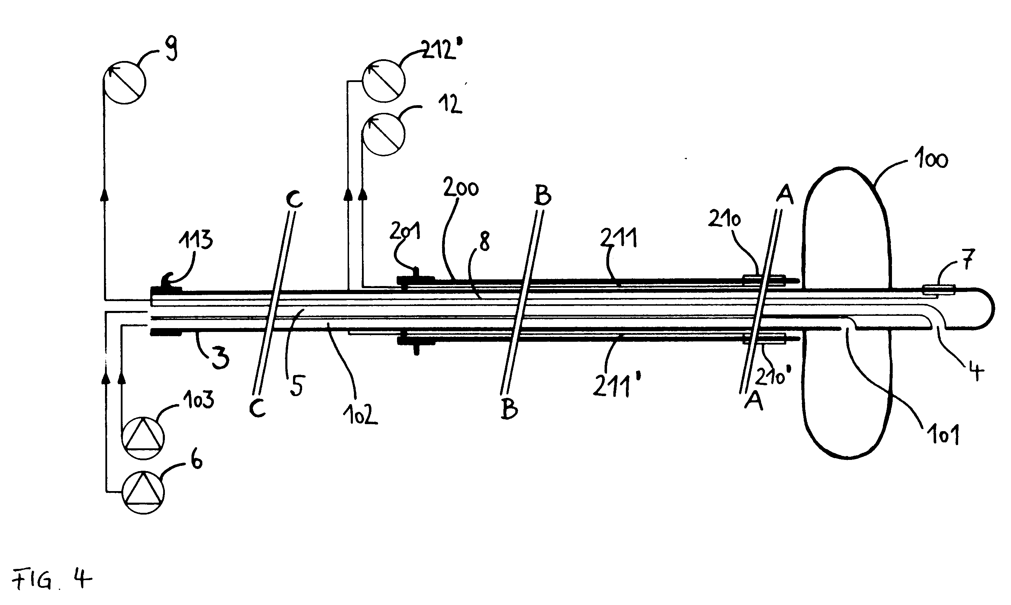

Figur 3 zeigt einen bekannten Katheter, mit dem eine Abdichtung der inneren Harnröhrenmündung möglich ist. Das Katheterrohr 3 ist hier im Prinzip gleich ausgebildet, wie das herkömmliche Katheterrohr gemäß Figur 1. Gleiche Merkmale sind mit gleichen Bezugzeichen bezeichnet. Neu ist, daß zwischen dem am Ende des Katheterrohres 3 angeordneten Druckaufnehmer 7 und dem im mittleren Bereich angeordenten Druckaufnehmer 10 auf dem Katheterrohr 3 ein Ballon 100 angeordnet ist. Dieser ist über eine durch das Katheterrohr 3 führende Leitung 102 mit einer Pumpe 103 verbindbar, von welcher aus ein Fluid, d.h. eine Flüssigkeit oder ein Gas in den Ballon 100 gepumpt werden kann, so daß dieser sich aufbläht und den inneren Mündungsbereich der Harnblase 2 (siehe Figur 1) gegen die Harnblase 1 hin abdichtet. Wegen des Ballons 100 kann der Katheter zur Aufnahme von Streßprofilen nicht mehr zurückgezogen werden. Um dennoch die Aufnahme eines Streßprofiles zu ermöglichen, sind in Längsrichtung des Katheterrohres 3 eine Reihe weiterer Druckaufnehmer 110a-110e angeordnet, die über durch das Katheterrohr führende Leitungen 111a-111e mit entsprechenden Geräten 112a 112e zur Messung und Aufzeichnung des Harnröhrendruckes verbunden sind. An dem außerhalb der Harnröhre bleibenden Ende des Katheterrohres 3 befindet sich ein Haken 113 zur federnden Befestigung des Katheters, wie dies später noch beschrieben wird.

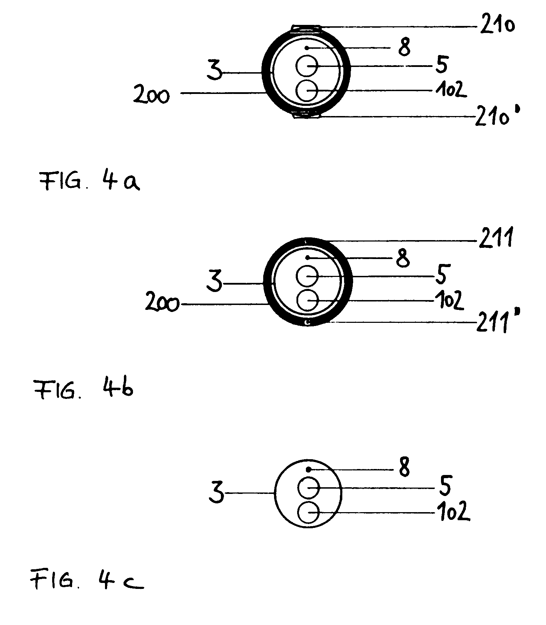

Figur 4 zeigt eine erste Ausführungsform des erfindungsgemäßen Katheters, wobei Elemente, die gleich sind wie bei dem Katheter nach Figur 1 oder Figur 3 mit den gleichen Bezugsziffern wie dort bezeichnet sind. Neu ist hier, daß auf dem Katheterrohr 3 ein kürzeres Mantelrohr 200 angeordnet ist, das das innere Katheterrohr 3 konzentrisch umgibt und auf diesem verschiebbar ist.

Am Einführende des Mantelrohres 200 sind diametral gegenüberliegend zwei Druckaufnehmer 210, 210' vorgesehen, mit denen beim Zurückziehen des Mantelrohres 200 das Harnröhrenprofil ermittelt werden kann. Die beiden Druckaufnehmer 210, 210' sind über in der Wandung des äußeren Mantelrohres 200 verlaufende elektrische Leitungen 211, 211' mit Meßgeräten 12, 212' verbunden, mit denen der Druck in der Harnröhre gemessen und aufgezeichnet werden kann. Auch hier ist wiederum zwischen dem am Ende des inneren Katheterrohres 3 angeordneten Druckaufnehmer 7 und den beiden am Ende des Mantelrohres 200 befindlichen Druckaufnehmern 210,210' auf dem inneren Katheterrohr 3 ein Ballon 100 angeordnet, der in der im Zusammenhang mit Figur 3 beschriebenen Weise gefüllt werden kann. Zu erwähnen ist noch, daß an dem außerhalb der Harnröhre verbleibenden Ende des Mantelrohres 200 ein Mitnehmerring angeordnet ist, der zum Rückzug des Mantelrohres dient, wie dies später noch erläutert wird.

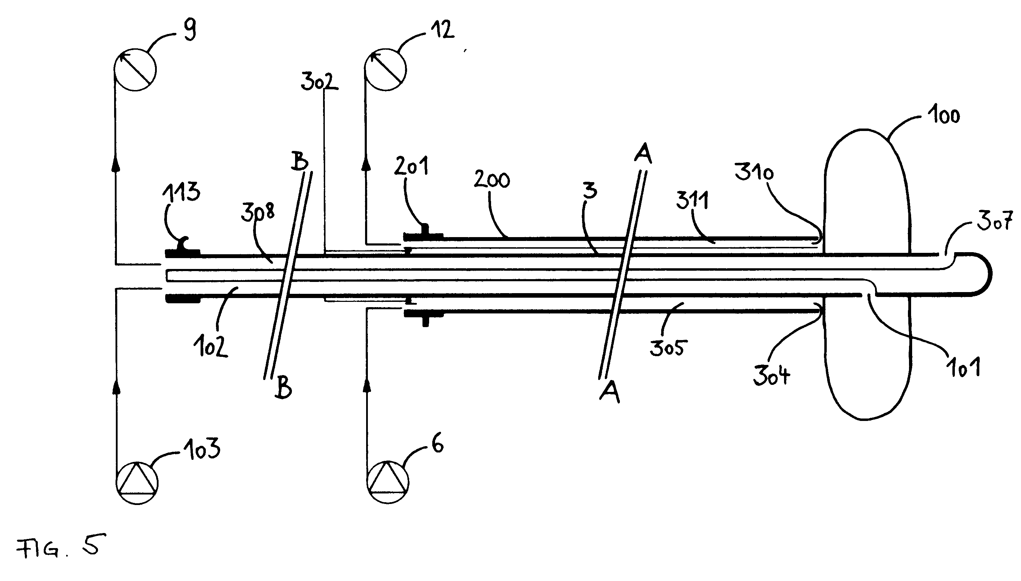

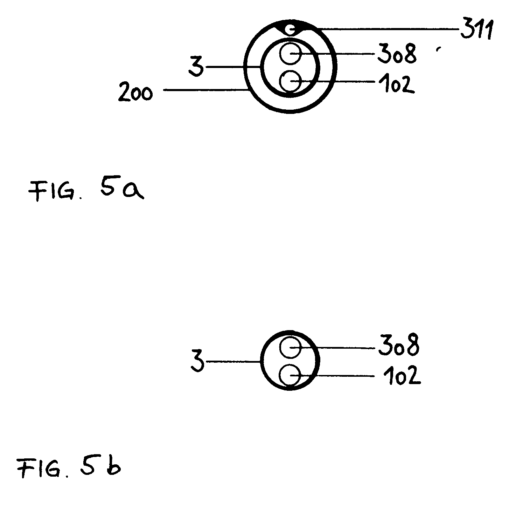

In Figur 5 ist eine zweite Ausführungsform des erfindungsgemäßen Katheters gemäß Figur 4 gezeigt. Auch hier sind gleiche Elemente wie bei den Kathetern gemäß den Figuren 1,3 und 4 wiederum mit gleichen Bezugsziffern wie dort bezeichnet. Der Katheter besteht hier wiederum aus einem inneren Katheterrohr 3 und einem darauf verschiebbar angeordneten äußeren Mantelrohr 200. Ferner ist auch hier nächst dem Ende des inneren Katheterrohres 3 ein durch das Katheterrohr füllbarer Ballon 100 zum Abdichten der inneren Harnröhrenmündung vorgesehen. Neu ist, daß der am Ende des inneren Katheterrohres 3 vorzusehende Druckaufnehmer von einer Öffnung 307 gebildet ist, die über eine durch das innere Katheterrohr 3 verlaufende Leitung 308 mit einem Meßgerät 9 zur Messung und Aufzeichnung des Harnblasendruckes verbunden ist. Neu ist ferner, daß der am Einführende des Mantelrohres 200 vorzusehende Druckaufnehmer ebenfalls als Öffnung 310 ausgebildet ist, die über eine zwischen dem inneren Katheterrohr 3 und dem äußeren Mantelrohr 200 verlaufende Leitung 311 mit einem Meßgerät 12 zur Messung und Aufzeichnung des Harnröhrendruckes verbunden ist. Um in die Harnblase 1 Flüssigkeit einführen zu können, ist hier zwischen dem äußeren Mantelrohr 200 und dem inneren Katheterrohr 3 eine weitere Leitung 305 vorgesehen, die am Einführende des äußeren Mantelrohres 200 in eine Öffnung 304 mündet. An dem anderen Ende ist die Leitung 305 mit einer Pumpe verbunden, welche die Flüssigkeit fördert, die in die Harnblase eingeleitet werden soll. Wenn das äußere Mantelrohr 200 zur Harnröhrendruckmessung zurückgezogen wird, so kann über die Leitung 305 und die Ausströmöffnung 304 Flüssigkeit nachgeliefert werden, welche den durch das Zurückziehen des Mantelrohres 200 in der Harnröhre freiwerdenden Raum ausgleicht. Die Leitung 305 dient demnach auch als Perfusionsleitung. Mit 302 ist ein Dichtungsring zwischen dem inneren Katheterrohr 3 und dem äußeren Mantelrohr 200 bezeichnet.

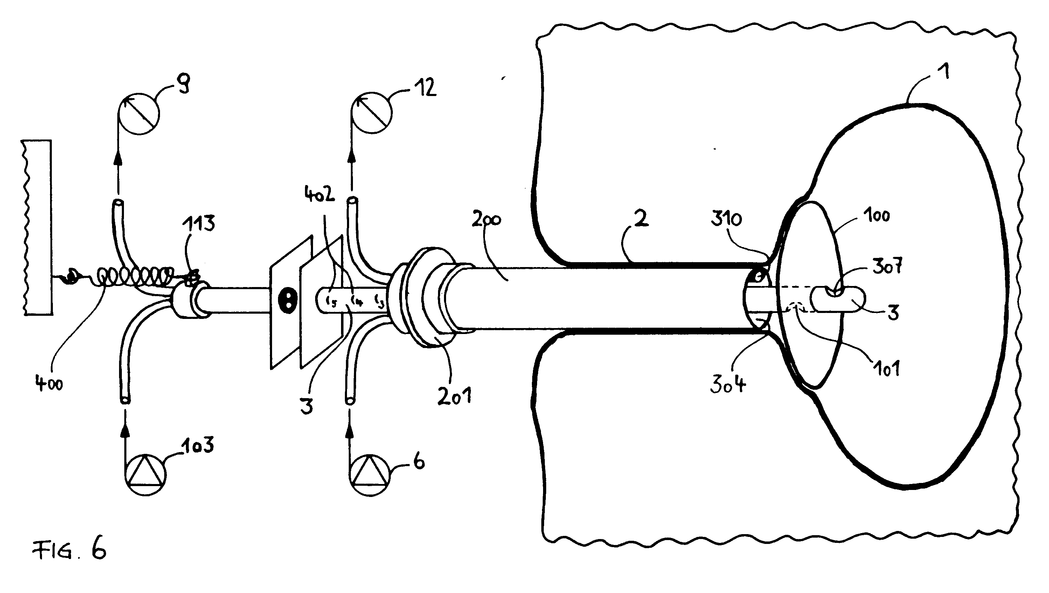

Figur 6 zeigt den in Figur 5 dargestellten Katheter schematisiert in perspektivischer Darstellung, eingeführt in die Harnblase 2 und die Harnröhre 1. Auch hier sind gleiche Elemente wiederum mit gleichen Bezugzeichen versehen worden. Neu gegenüber Figur 5 ist eine auf dem inneren Katheterrohr 3 vorgesehene Skala 402, an welcher der Rückzugsweg des äußeren Mantelrohres 200 durch die Harnröhre 2 gegenüber dem inneren Katheterrohr 3 abgelesen werden kann. Neu ist ferner, daß mittels einer Zugfeder 400 auf das innere Katheterrohr 3 eine Zugspannung ausgeübt wird, die den aufgeblähten Ballon 100 gegen die innere Harnröhrenmündung preßt und dadurch sicher abdichtet, und zwar unabhängig von der Lage, in der sich die Patientin bei der Untersuchung befindet. Die Zugfeder 400 ist einerseits ortsfest und andererseits an dem am äußeren Ende des inneren Katheterrohres 3 befindlichen Haken 113 befestigt.

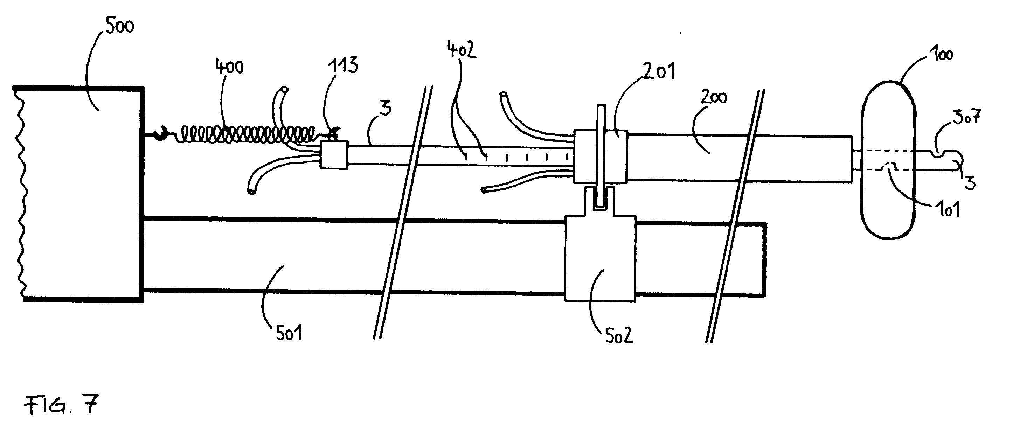

Figur 7 zeigt eine Rückzugseinrichtung, mit der ein zeitlich steuerbarer Rückzug des äußeren Mantelrohres 200 gegenüber dem inneren Katheterrohr 3 möglich ist. Mit 500 ist ein ortsfestes Motorengehäuse bezeichnet, an dem hier die Zugfeder 400 mit einem Ende befestigt ist. Das andere Ende der Zugfeder 400 greift wiederum an dem Haken 113 am äußeren Ende des inneren Katheterrohres 3 an. Von dem Motorengehäuse 500 geht eine Trägerschiene 501 aus, auf der ein Mitnehmer 502 verschiebbar angeordnet ist. Der Mitnehmer steht mit dem am äußeren Ende des Mantelrohres 200 vorgesehenen Mitnehmerring 201 in Eingriff. Innerhalb der Trägerschiene 501 verläuft eine nicht sichtbare Spindel, die von dem ebenfalls nicht sichtbaren Motor im Gehäuse 500 angetrieben wird. Die Spindel läuft in einer mit dem Mitnehmer 502 verbundenen Spindelmutter mit der Folge, daß der Mitnehmer 502 bei Drehung der Spindel zurückgezogen wird. Es ist natürlich auch möglich, die ganze Trägerschiene 501 zurückzuziehen, beispielsweise, wenn sie als Zahnstange ausgebildet ist und mit einemvon dem Motor angetriebenen Zahnrad kämmt.

**Patentansprüche**

1. Katheter zur Blasen- und Harnröhrendruckmessung beim Menschen, insbesondere bei Frauen, bestehend aus einem Katheterrohr (3) mit einem ersten Druckaufnehmer (7,307) an seinem in die Harnblase (1) einzuführenden Ende, mit mindestens einem zweiten Druckaufnehmer (210,210';310) in seinem mittleren Bereich, welcher nach Einführen des Katheterrohres (3) in der Harnröhre (2) zu liegen kommt, und mit einem an dem Katheterrohr (3) vorgesehenen Mittel zum Abdichten der inneren Harnröhrenmündung zwischen dem ersten und dem zweiten Druckaufnehmer,
**dadurch gekennzeichnet,**
daß das Katheterrohr (3) von einem gegenüber diesem verschiebbaren Mantelrohr (200) umgeben ist, an dessen Ende sich der zweite Druckaufnehmer (210,210';310) befindet.(Fig.4-7)

2. Katheter nach Anspruch 1,
**dadurch gekennzeichnet,**
daß das Mittel zum Abdichten der Harnröhrenmündung von einem auf dem Katheterrohr (3) angeordneten und über dieses von außen mit einem Fluid füllbaren Ballon (100) gebildet ist. (Fig.4-7)

3. Katheter nach Anspruch 1 oder 2,
**dadurch gekennzeichnet,**
daß am Ende des Mantelrohres (200) in Rohrumfangsrichtung mindestens zwei Druckaufnehmer angeordnet sind. (Fig.4)

4. Katheter nach einem der vorhergehenden Ansprüche,
**dadurch gekennzeichnet,**
daß die Druckaufnehmer von Mikrotransducern (7,10,110a-110e,210) gebildet sind, deren Anschlußleitungen (8,11,111a-111e,211,211') durch den Katheter nach außen geführt und mit Druckmeßgeräten (9,12) verbindbar sind. (Fig.4)

5. Katheter nach einem der vorhergehenden Ansprüche,
**dadurch gekennzeichnet,**
daß die Druckaufnehmer von Öffnungen (307,310) gebildet sind, die den Ausgang von durch den Katheter nach außen geführte Leitungen (308,311) bilden, welche mit äußeren Druckmeßgeräten (9,12) verbindbar sind.(Fig.5-7)

6. Katheter nach Anspruch 1 oder 2,
**dadurch gekennzeichnet,**
daß am Ende des Mantelrohres (200) eine Öffnung (304) vorgesehen ist, die den Ausgang einer durch das Mantelrohr (200) geführten Leitung (305) bildet, welche mit einer Pumpe (6) zum Einführen von Füssigkeit in die Harnblase (1) oder zur Perfusion von Flüssigkeit in die Harnröhre (2) verbindbar ist. (Fig.5-7)

7. Katheter nach einem der vorhergehenden Ansprüche,
**dadurch gekennzeichnet,**
daß eine Spanneinrichtung vorgesehen ist, mittels welcher das das Abdichtmittel tragende Katheterrohr (3) unter eine Spannung setzbar ist, die die Tendenz hat, das Katheterrohr (3) aus der Harnblase (1) herauszuziehen und damit das Abdichtmittel gegen die innere Harnröhrenmündung zu pressen. (Fig.6 und 7)

8. Katheter nach Anspruch 7,
**dadurch gekennzeichnet,**
daß die Spanneinrichtung von einer Zugfeder (400) gebildet ist, die mit ihrem einen Ende ortsfest verankerbar und mit ihrem anderen mit dem Katheterrohr (3) verbindbar ist. (Fig. 6 und 7)

9. Katheter nach Anspruch 1 oder 2,
**dadurch gekennzeichnet,**
daß eine Rückzugseinrichtung zum Zurückziehen des Mantelrohres (200) gegenüber dem Katheterrohr (3) vorgesehen ist, mittels welcher das Katheterrohr (3) weitgehend ortsfest aber elastisch fixierbar ist und mittels welcher ferner das Mantelrohr (200) motorisch entsprechend einem vorgegebenen Zeitverlauf gegenüber dem Katheterrohr (3) in der Harnröhre (2) zurückziehbar ist. (Fig. 7)

## Claims

1. A catheter for measuring the pressure in the bladder and urethra of human beings, in particular of women, comprising a catheter tube (3) having a first pressure sensor (7,307) at its end to be inserted into the bladder (1), at least one second pressure sensor (210,210';310) in its middle region which, after the catheter tube (3) has been inserted into the bladder (3), lies in the urethra (2), and means provided on the catheter tube (3) for sealing the inner opening of the urethra between the first and the second pressure sensors, characterised in that the catheter tube (3) is surrounded by a tubular jacket (200) displaceable relative thereto and having the second pressure sensor (210,210';310) located at its end. (Figs.4-7)

2. A catheter according to claim 1, characterised in that the means for sealing the opening of the urethra comprises a balloon (100) that is arranged on the catheter tube (3) and can be filled therethrough with a fluid from outside. (Figs. 4-7)

3. A catheter according to claim 1 or claim 2, characterised in that at least two pressure sensors are arranged at the end of the tubular jacket (200) in the peripheral direction of the tube. (Fig. 4)

4. A catheter according to any one of the preceding claims, characterised in that the pressure sensors comprise micro-transducers (7,10,110a-110e,210) whose leads (8,11,111a-111e,211,211') pass through the catheter to the exterior and can be connected to pressure measuring devices (9, 12). (Fig. 4)

5. A catheter according to any one of the preceding claims, characterised in that the pressure sensors comprise openings (307,310) which form the outlet for the leads (308,311) pass through the catheter to the exterior and can be connected to external pressure measuring devices (9, 12). (Figs. 5-7)

6. A catheter according to claim 1 or claim 2, characterised in that an opening (304) is provided at the end of the tubular jacket (200) that forms the outlet of a conduit (305) guided through the tubular jacket (200) that can be connected to a pump (6) for introducing fluid into the bladder (1) or for perfusion of fluid into the urethra (2). (Figs. 5-7)

7. A catheter according to any one of the preceding claims, characterised in that a tensioning device is provided by means of which the catheter tube (3) carrying the sealing means can be subjected to tension which tends to pull the catheter tube (3) out of the bladder (1) and thereby presses the sealing means against the inner urethral opening. (Figs. 6 and 7)

8. A catheter according to claim 7, characterised in that the tensioning device comprises a tension spring (400) that can be lodged immovably at its one end and can be connected at its other end to the catheter tube (3). (Figs. 6 and 7).

9. A catheter according to claim 1 or claim 2, characterised in that a pull-back device is provided for pulling back the tubular jacket (200) relative to the catheter tube (3) by means of which the catheter tube (3) can be affixed substantially immovably but elastically and by means of which the tubular jacket (200) can also be pulled back relative to the catheter tube (3) into the urethra (2) by a motor according to a predetermined function of time. (Fig. 7)

**Revendications**

1. Catheter pour la mesure de la pression à l'intérieur de la vessie et de l'urètre chez l'être humain, en particulier chez la femme, composé d'un tube de catheter (3) comportant un premier capteur de pression (7, 307) à son extrémité à introduire dans la vessie (1), au moins un deuxième capteur de pression (210, 210'; 310) dans sa région médiane qui, après introduction du tube de catheter (3), est situé dans l'urètre (2) et un moyen prévu sur le tube de catheter (3) pour assurer l'étanchéité au niveau du débouché interne de l'urètre, entre le premier et le deuxième capteur de pression, caractérisé en ce que le tube de catheter (3) est entouré d'un tube-enveloppe (200) qui peut coulisser par rapport à lui et à l'extrémité duquel se trouve le deuxième capteur de pression (210, 210'; 310). (figure 4-7)

2. Catheter selon la revendication 1, caractérisé en ce que le moyen pour assurer l'étanchéité au niveau du débouché de l'urètre est formé par un ballon (100) qui est disposé sur le tube de catheter (3) et qui peut être rempli au moyen d'un fluide à partir de l'extérieur par l'intermédiaire dudit tube de catheter. (figure 4-7)

3. Catheter selon la revendication 1 ou 2, caractérisé en ce qu'au moins deux capteurs de pression sont disposés à l'extrémité du tube enveloppe (200) dans la direction tangentielle.

4. Catheter selon l'une des revendications précédentes, caractérisé en ce que les capteurs de pression sont formés par des microtransducteurs (7, 10, 110a-100e, 210) dont les lignes de connexion (8, 11, 111a-111e, 211, 211') sont amenées à l'extérieur à travers le catheter et peuvent être reliées aux appareils de mesure de la pression (9, 12). (figure 4)

5. Catheter selon l'une des revendications précédentes, caractérisé en ce que les capteurs de pression sont formés par des ouvertures (307, 310) qui constituent la sortie de lignes (308, 311) qui sont amenées vers l'extérieur à travers le catheter et qui peuvent être reliées à des appareils de mesure de la pression extérieurs (9, 12). (figure 5-7)

6. Catheter selon la revendication 1 ou 2, caractérisé en ce qu'il est prévu, à l'extrémité du tube enveloppe (200), une ouverture (304) qui constitue la sortie d'un tube (305) qui traverse le tube enveloppe (200) et qui peut être relié à une pompe (6) pour l'introduction de liquide dans la vessie (1) ou pour la perfusion de liquide dans l'urètre (2). (figures 5-7)

7. Catheter selon l'une des revendications précédentes, caractérisé en ce qu'il est prévu un dispositif de tension à l'aide duquel une tension peut être appliquée sur le tube de catheter (3) portant le moyen d'étanchéité, laquelle tension a tendance à tirer le tube de catheter (3) hors de la vessie (1) et ainsi, à presser le moyen d'étanchéité contre le débouché interne de l'urètre. (figures 6 et 7)

8. Catheter selon la revendication 7, caractérisé en ce que le dispositif de tension est constitué d'un ressort de traction (400) qui peut être attaché fixement par l'une de ses extrémités et est relié au tube de catheter (3) par son autre extrémité. (figures 6 et 7)

9. Catheter selon la revendication 1 ou 2, caractérisé en ce qu'il est prévu un dispositif de traction pour tirer le tube-enveloppe (200) par rapport au tube de catheter (3) au moyen duquel le tube de catheter (3) peut être attaché sensiblement fixement mais élastiquement et au moyen duquel, en outre, le tube-enveloppe (200) peut être retiré mécaniquement par rapport au tube de catheter (3) dans l'urètre (2) selon un diagramme de temps prédéterminé. (figure 7)

FIG. 1

EP 0 258 690 B1

FIG. 2 a

FIG. 2 b

FIG. 3

EP 0 258 690 B1

110 b

111a

11

8

3

5

102

FIG. 3a

11, 111a - 111e

8

3

5

102

FIG. 3b

FIG. 4

EP 0 258 690 B1

EP 0 258 690 B1

210

8

3

200

5

102

210'

**FIG. 4a**

211

8

3

200

5

102

211'

**FIG. 4b**

8

3

5

102

**FIG. 4c**

FIG. 5

EP 0 258 690 B1

FIG. 5a

FIG. 5b

EP 0 258 690 B1

FIG. 6

FiG. 7